# EUROPEAN PATENT APPLICATION

(11) **EP 1 384 787 A1**
(43) Date of publication of application: **28.01.2004**
(21) Application number: 02016336.6
(22) Date of filing: 25.07.2002
(51) Int. Cl.: C12Q 1/48, G01N 33/50, A01K 67/033

(54) **Screening method for the identification and characterization of DNA methyltransferase inhibitors**

(71) Applicant: Deutsches Krebsforschungszentrum Stiftung des öffentlichen Rechts, 69120 Heidelberg (DE)
(72) Inventor: Lyko, Frank, 69115 Heidelberg (DE)
(74) Representative: Isenbruck, Günter, Dr.

(57) **Abstract**

The present invention therefore provides a screening method for the identification and characterization of inhibitors of DNA methyltransferases comprising the following steps:
a) generation of at least one *Drosophila* culture
   which culture comprises at least one offspring that is derived from a cross between flies of the *Drosophila* strains *GawB(69B)* and *UAS-Dnmt3a* and in an appropriate developmental stage,
   a sufficient amount of *Drosophila* medium
   and an appropriate concentration of at least one candidate compound for a DNA methyltransferase inhibitor,
b) incubation of the the *Drosophila* culture of step a) under incubation conditions which allow normal *Drosophila* development for an appropriate incubation period,
c) identification of those *Drosophila* cultures of step b) that show the presence of mobile larvae or pupae.

In another aspect, the invention concerns the offsprings derived from a cross between the transgenic *Drosophila* strains *GawB(69B)* and *UAS-Dnmt3a*, as well as their use for the identification and characterization of inhibitors of DNA methyltransferases, particularly of inhibitors of Dnmt3a.

## Description

In one aspect, the present invention concerns a screening method for the identification and characterization of inhibitors of DNA methyltransferases by culturing *Drosophila* offsprings which are derived from a cross between flies of the *Drosophila* strains *GawB(69B)* and *UAS-Dnmt3a* in the presence of a candidate compound for a DNA methyltransferase inhibitor for an appropriate time and by subsequent identification of those *Drosophila* cultures that do not show premature lethality at the prepupal developmental stage.

In another aspect, the invention relates to the use of the offsprings from a cross between the transgenic *Drosophila* strains *GawB(69B)* and *UAS-Dnmt3a* for the identification and characterization of inhibitors of DNA methyltransferases, particularly of inhibitors of the DNA methyltransferase Dnmt3a.

It has been known for a long time that tumour development does not only coincide with genetic mutations, but also with epigenetic mutations. Those epigenetic mutations involve changes in the DNA methylation pattern and modifications of specialized chromatin structures which are capable of stably modulating gene expression over many cell generations. Thus, an epigenetic mutation can have the same effect like a classical, genetic mutation, i.e. a loss-of-function or gain-of-function of a given gene.

The modifications in DNA methylation during tumourigenesis triggered a considerable interest in the enzyme family of DNA methyltransferases.

DNA methyltransferases are central factors in epigenetic gene regulation and mammalian methyltransferases have been analyzed extensively. All known (cytosine-5) DNA methyltransferases, from bacteria to man, share a common structure: Their C-terminal domain contains defined catalytic signature motifs that are required for enzymatic methyltransferase function (Kumar et al., Biochemistry, 31, p. 8648- 8653, 1992). In addition, most eukaryotic DNA methyltransferases also contain an N-terminal "regulatory" domain that can mediate various functions, mostly by protein-protein interactions.

Presently, there are 4 known human DNA methyltransferases: Dnmt1, Dnmt2, Dnmt3A and Dnmt3B. Dnmt1 has been shown to be essential for mammalian development from the onset of cellular differentiation (Li et al., Cell, 69, p. 915 - 926, 1992). The enzyme has been analyzed in high detail, both *in vitro* and *in vivo.* Most *in vivo* results are consistent with Dnmt1 encoding a pure maintenance methyltransferase (Lyko et al., Nat. Genet., 23, p. 363- 366, 1999) that is responsible for copying methylation patterns from the parental DNA strand to the newly replicated daughter strand. Dnmt1 contains a very large regulatory domain that mediates protein-protein interactions with several factors involved in DNA replication, cell cycle control and chromatin structure.

Further human DNA methyltransferases have been discovered more recently and are now being characterized. The function of Dnmt2, the second known DNA methyltransferase, is still far from being understood (Okano et al., Nucl. Acid Res., 26, p. 2536- 2540, 1998). Unlike most other eukaryotic DNA methyltransferases, Dnmt2 does not contain a regulatory domain and therefore looks similar to bacterial methyltransferases. Although the enzyme contains all the catalytic consensus motifs, no enzymatic activity could be detected yet. Dnmt2 is highly conserved during evolution with known homologs in a variety of diverse organisms such as yeasts, insects, plants and vertebrates (Dong et al., Nucl. Acid Res., 29, p. 439- 448, 2001).

The most recently discovered DNA methyltransferases, Dnmt3A and Dnmt3B, are two closely related enzymes that have been demonstrated to function as *de novo* methyltransferases *in vitro* and *in vivo* (Hsieh et al., Mol. Cell Biol., 19, p. 8211 - 8218, 1999; Lyko et al., Nat. Genet., 23, p. 363- 366, 1999; Okano et al., Cell 99, p. 247- 257, 1999). Both enzymes are essential for mammalian development as demonstrated by gene targeting in mice. While the target genes of Dnmt3A are still unknown, the function of Dnmt3B appears to be restricted to methylation of centromeric satellite repeats (Okano et al., Cell, 99, p. 247- 257, 1999). This particular sequence specificity also linked Dnmt3B to ICF syndrome, a rare autosomal recessive disorder characterized by immunodeficiency, centromeric instability and facial anomalies. Loss-of-function mutations in the Dnmt3B gene have now been identified in a considerable number of ICF syndrome patients, thus revealing the molecular origin of the disease (Hansen et al., Proc. Natl. Acad. Sci., 96, p. 14412 - 14417, 1999; Okano et al., Cell, 99, p. 247- 257, 1999).

DNA methyltransferase expression has been analyzed in a variety of tumours. Cells from tumour tissues show a significantly modified DNA methylation pattern compared to cells from healthy tissue. Particularly, it could be shown that cells of specific tumour types show a hypermethylation of their genomic DNA. *De novo* DNA methyltransferases like Dnmt3a and Dnmt3b are capable of methylating DNA directly and are therefore very likely to contribute to the hypermethylation of tumour genomes.

Apart from the indications for an accumulation of epigenetic mutations during tumourigenesis there are additional lines of experimentation that provide direct proof for a role of DNA methylation in cancer development.

For example, it has been shown that genomic demethylation greatly reduces the number of intestinal polyps in the Apc^{Min} mouse strain (Laird et al., Cell, 81, p. 197 - 205, 1995). This mouse strain represents an excellent model system for colorectal cancer since it carries a mutation in the *Apc* gene that causes the formation of numerous intestinal polyps and tumours. The incidence of lesions was significantly decreased by either mutational inactivation of the *Dnmt1* gene or by a pharmacological inhibition of DNA methyltransferase or by a combination of both (Laird et al., Cell, 81, p. 197 - 205, 1995). Genomic demethylation therefore reverted epimutations that normally facilitate the generation of intestinal polyps caused by the *Apc* mutation.

The therapeutic potential of genomic demethylation has also been demonstrated in human cancer cells. Melanoma cells, for example, frequently show a high degree of resistance to conventional chemotherapeutic agents. This chemoresistance could be overcome by treatment of cells with an inactivator of DNA methyltransferases (Soengas et al., Nature, 409, p. 207- 211, 2001). The effect is caused by reversion of the methylation-induced silencing of the apoptosis effector gene *Apaf-1.* Experimental demethylation therefore reverted an epigenetic mutation in the apoptosis pathway that had rendered the cells incapable of responding to signals triggered by chemotherapeutic agents.

The significance of epigenetic mutations for cancer therapy lies in their reversibility. In contrast to genetic mutations that can only be treated by removal of affected tissue, epigenetic mutations can be reverted. Reversion results in epigenetic reprogramming or resetting from the epigenotype of tumour cells to the epigenotype of normal cells. In this respect, inhibitors of DNA methyltransferases have been used with some limited success.

The most widely known inhibitor is the cytidine analogue 5-aza-cytidine and it has been used in the vast majority of experiments where pharmacological inhibition of methyltransferase activity is involved. The compound gets incorporated into DNA and functions as a suicide analogue for DNA methyltransferases (Santi et al., Proc. Natl. Acad. Sci., 81, p. 6993- 6997, 1984). The DNA methyltransferases recognize 5-aza-cytidine as a natural substrate and initiate catalysis. However, with the inhibitor, a covalent reaction intermediate between substrate and enzyme cannot be resolved and the enzyme remains bound to DNA (Santi et al., Proc. Natl. Acad. Sci., 81, p. 6993- 6997, 1984). The toxicity of these protein-DNA adducts probably accounts for the highly toxic effects of 5-aza-cytidine. The toxicity of 5-aza-cytidine also presented a major obstacle in attempts to use the inhibitor in cancer therapy. Low concentrations of 5-aza-cytidine have been used in clinical trials (Abele et al., Eur. J. Cancer Clin. Oncol., 23, p. 1921 - 1924, 1987; Lubbert et al., Curr. Top. Microbiol. Immunol. 249, p. 135 - 164, 2000; Pinto et al., Leukemia, 7 (Suppl. 1), p. 51- 60, 1993), but the success of these experiments has been rather limited.

Several strategies are now being pursued to establish a more effective concept for epigenetic cancer therapy. For example, chemical compounds, particularly molecules with low molecular weight ("small molecules"), could be identified that inhibit DNA methyltransferases directly without being incorporated into DNA. Small molecules with these inhibitor properties could be effective in cancer therapy, but might show a marked reduction in toxicity compared to known DNA methyltransferase inhibitors as 5-aza-cytidine.

However, screening for inhibitors of DNA methyltransferases is a complex task, because *in vivo* assays with an easy experimental read-out are not available.

The only *in vivo* sceening assays for the characterization of candidate inhibitors of DNA methyltransferases known so far have been established in cell culture or in mice. In these *in vivo* screening assays the candidate inhibitors are added to the cell culture medium or were injected directly into the test mice. After several weeks of incubation time genomic DNA is isolated from the cultured cells or from mouse tissue for the molecular genetic analysis of modifications of the DNA methylation pattern (Siegmund and Laird, Methods 27, p.170-178, 2002). Since the performance of these screening assays takes several weeks and requires work-intensive and expensive incubation and analysis steps, like long-term culturing of cells, DNA isolation and analysis of the DNA methylation pattern, these *in vivo* screening assays in mice or in cultured cells do not allow the parallel analysis of a large number of candidate DNA methyltransferase inhibitors in a high-throughput screen.

The known *in vitro* test systems to identify DNA methyltransferase inhibitors also require labourious analysis steps (Tamura et al., Nat. Prod. Lett. 16, p. 25-27, 2002). Moreover, *in vitro* screening assays for the identification of DNA methyltransferase inhibitors have the additional disadvantage that they are based on bacterial methyltransferases and do not allow significant *in vivo* conditions, like the influence of tumour relevant parameters, to be considered during the analysis. In so far, the results of *in vitro* assays for the identification of new DNA methyltransferase inhibitors should have a rather limited relevance for cancer research.

It is an object of the present invention to provide a high-throughput screening method for the identification and characterization of inhibitors of DNA methyltransferases performed under *in vivo* conditions, which allows the parallel screening of a large number of candidate inhibitors.

This object is achieved by an efficient screening method for the identification and characterization of inhibitors of DNA methyltransferases comprising the following steps:
a) generation of at least one *Drosophila* culture
   which culture comprises at least one offspring that is derived from a cross between flies of the *Drosophila* strains *GawB(69B)* and *UAS-Dnmt3a* in an appropriate developmental stage, a sufficient amount of *Drosophila* medium and an appropriate concentration of at least one candidate compound for a DNA methyltransferase inhibitor,
b) incubation of the the *Drosophila* culture of step a) under conditions which allow normal *Drosophila* development for an appropriate incubation period,
c) identification of those *Drosophila* cultures of step b) that show the presence of mobile larvae or pupae.

The *Drosophila* screening assay according to the present invention allows the parallel screening of a large number of chemical compounds, e.g. several hundreds of chemical compounds (for instance from a library of small molecules) within a short time. Furthermore the assay requires no labourious, expensive or time-consuming analysis steps. Thus, this screening method allows the parallel high-throughput screening of a large number of candidate inhibitors, but still is performed under *in vivo* conditions. By this, the screening method of the invention combines all required advantages and overcomes the deficits of the other aforementioned screening methods known from the prior art.

The screening system of the present invention makes use of the *Drosophila* model system, which is a rather simple model organism and therefore combines numerous advantages compared to other model organisms. Particularly, in *Drosophila* most of the significant molecular mechanisms are identical to the corresponding mechanisms in humans. Thus, on the one hand, the results of a DNA methyltransferase-inhibitor screening assay in *Drosophila* should also be relevant for cancer therapy in humans. On the other hand, in contrast to the mouse model or other complex model organisms, large numbers of the fruit fly *Drosophila* can be maintained within very limited space, their culture being highly cost-efficient and the generation time required being only 10 days, on the average.

Another crucial advantage of the *Drosophila* model system for the establishment of a screening method to detect DNA methyltransferase inhibitors can be seen in the negligible activity of the endogenous DNA methylation system in *Drosophila.* Therefore the effect of a recombinantly expressed mammalian DNA methyltransferase can be easily studied and characterized in *Drosophila* without being confused with the effects of endogenous DNA methyltransferases.

For cultivation of *Drosophila,* generally several flies are put into a culture vial with *Drosophila* medium. These vials are then kept in a standard incubator at 20 - 30°C, particularly at 25°C. The relative humidity is kept generally at 50 to 70 %, particularly at 60 % relative humidity. Female flies will start laying eggs onto the food immediately. After one day, larvae hatch from the eggs. Larval development takes place in the culture medium and involves three consecutive stages, the first, second and third larval stage, each lasting for about one day. At the end of the third larval stage, larvae crawl out of the medium and pupariate on the wall of the culture vial. Normal pupal development requires about 3 additional days and ends with the eclosion of an adult fly. Adult flies reach sexual maturity within a few hours and females start laying eggs about 2 days after fertilization.

The screening method of the present invention makes use of a new transgenic *Drosophila* strain, which carries a genomically integrated expression system comprising a combination of two transgenic constructs. This new transgenic *Drosophila* strain can be produced by crossing flies of the transgenic strain *GawB(69B)* (the so called "inducer strain") and flies of the transgenic strain *UAS-Dnmt3a* (the so-called "responder strain").

The "responder strain" *UAS-Dnmt3a* has been produced by the integration of an expression cassette comprising an uptream activating sequence (UAS) which is a binding site for the transcription factor Gal4 followed by the coding sequence of the Dnmt3a gene and the "mini-white" gene which has been used as a selection marker indicating a successful integration of the transgenic expression cassette.
The "responder strain" *UAS-Dnmt3a* as well as its generation has been previously described in Lyko et al, Nature Genetics, 23, p. 363- 366 (1999). In this publication the crossings of the "responder strain" *UAS-Dnmt3a* with two other "inducer strains" have been disclosed, one leading to the phenotype of lethality in the developmental stage of pupariation, the other leading to the phenotype of an impaired eye development.

The "inducer strain" *GawB(69B)* has been produced as previously described in Brand et al, Development 118, p. 401- 415 (1993). To generate different transgenic *Drosophila* strains each expressing Gal4 in a tissue- and cell-specific manner, the Gal4 gene has been inserted randomly into the genome, driving Gal4 expression from numerous different genomic enhancers. Time- and tissue-specifity of the expression of the transgenic Gal4 gene is thereby dependent on the location of the integration of the Gal4 gene into the genome. One of the various transgenic *Drosophila* strains that was maintained in the described way is the "inducer strain" *GawB(69B)* that expresses the Gal4 gene under the control of an endogenous regulatory element at a specific time in development, in specific tissues at a specific level. The expression level and time of Gal4 thereby depends on the proximity of endogenous regulatory elements, i.e. enhancer elements, to the integration site of the GAL4 gene in the *Drosophila* genome. The inducer strain *GawB(69B)* has been deposited in the *Bloomington Drosophila stock center* under reference no. 1774 (http://flybase.bio.indiana.edu/exec+:-s1774_vtext/html_-dR457109-457452-/.data/stocks /stock-centers/bloomington/bloomington. rpt:/.bin/fbstor.html).

The double transgenic offsprings of crossings between the mentioned transgenic *Drosophila* strains, *GawB(69B)* and *UAS-Dnmt3a,* that carry both transgenes, allow the tissue-specific overexpression of the mouse DNA methyltransferase *Dnmt3a* at a defined time during fly development. This specific genotype established by crossing the transgenic *Drosophila* strains *GawB(69B)* and *UAS-Dnmt3a* leads to lethality in the larval stage which is a phenotype that has not been described before.

The screening method according to the present invention can be used to identify and characterize inhibitors of all members of the enzyme family of DNA methyltransferases. The method is particularly appropriate to identify and characterize inhibitors of at least one DNA methyltransferase selected from the group consisting of Dnmt1, Dnmt2, Dnmt3a and Dnmt3b, preferably to identify and to characterize inhibitors of the DNA methyltransferase Dnmt3a .

In step a) of the screening method of the present invention, at least one *Drosophila* culture is generated which comprises at least one offspring that is derived from a cross between flies of the *Drosophila* strains *GawB(69B)* and *UAS-Dnmt3a* in an appropriate developmental stage, a sufficient amount of *Drosophila* medium and an appropriate concentration of at least one candidate compound for a DNA methyltransferase inhibitor.

Each of the cultures of step a) comprise at least one offspring, preferably more than 10, more preferably more than 50, most preferably 50 to 100 offsprings of the aforementioned cross.

Furthermore, it is preferred that each of cultures generated in step a) comprise equal numbers of offsprings. Generally the number of individual *Drosophila* cultures depends on the number of different candidate compounds or mixtures thereof and on the different concentrations of candidate compounds or mixtures thereof to be tested in the screen.

Generally the offsprings of step a) can be added to the individual cultures in any appropriate developmental stage that is prior to the stage of pupariation. For instance, the offsprings of step a) can be generated in the individual cultures of step a) as egg that is laid by female flies of the parental strains *GawB(69B)* and *UAS-Dnmt3a,* as freshly hatched larvae or as larvae in the first, second or third developmental stage.

Preferably the offsprings of step a) are added to or generated in the individual cultures as eggs which are laid by females of the parental fly strain *GawB(69B)* fertilized by flies of the strain *UAS-Dnmt3a* or as eggs which are laid by females of the parental fly strain *UAS-Dnmt3a* fertilized by flies of the strain *GawB(69B).* For the addition or generation of those Drosophila eggs in the individual cultures preferably a number of about 5 to 10 fertilized flies of the fly strains *GawB(69B)* and *UAS-Dnmt3a* are added to the individual cultures. The fertilized female flies will start to lay their eggs onto the food immediately.

The number of eggs laid in each of the individual cultures generally depends on the number of flies of the parental strains that were added. Generally at least one egg, preferably more than 10 eggs, more preferably more than 50 eggs, most preferably 50 to 100 eggs will be laid in each of the *Drosophila* cultures resulting in the corresponding number of offsprings.

Each of the cultures of step a) additionally comprise a sufficient amount of *Drosophila* medium that contains ingredients which are known to a person skilled in the art and that is commercially available. The amount of *Drosophila* medium that should be used to provide sufficient food for a specific number of flies is known to a person skilled in the art.

Furthermore, each of the cultures generated in step a) of the screening method of the present invention also comprise an appropriate concentration of at least one candidate compound for a DNA methyltransferase inhibitor to generate an intense contact between the developing flies and the tested candidate compound.

The individual candidate compounds for methyltransferase inhibitors are preferably dissolved in water, more preferably dissolved in *Drosophila* medium and are subsequently added to the each of the individual cultures of step a).

Furthermore, it is preferred that each of the individual candidate compounds for DNA methyltransferase inhibitors is screened in more than one concentration. The individual candidate compounds are therefore added in at least one appropriate concentration to the individual aliquots, preferably in concentrations from 100 µM to 10 mM.

As a candidate compound there can be used a single compound or a mixture of two or several compounds, in particular a library.

Generally all chemical compounds of interest can be used as candidate compounds to be tested for their DNA methyltransferase inhibitor activity. These chemical compounds of interest can be generated by various methods known to the art, preferably synthetically, in particular by combinatoric chemistry, or by biochemical methods, in particular by recombinant recombination or purification from biological probes.

In a preferred enbodiment of the present invention, the compound is of low molecular weight ("small molecules") or the library is composed of molecules with low molecular weight ("small molecule library").

A "small molecule" is defined as a complex collection of compounds, which are produced in a non-biological way, that means which are not produced by recombinant expression, like for instance most protein or peptide libraries. "Small molecules" can be generated by various methods known to the art, but are preferably produced by synthetically, more preferably by combinatoric chemistry, to generate a compound library with a maximum chemical diversity within the constraints of predicted attractive drug characteristics.

In another preferred enbodiment of the present invention, the compound is water-soluble or is composed of water-soluble chemical compounds, in particular the compound is a protein or peptide or a library thereof. Proteins, peptides or libraries composed of proteins or peptides can be generated by various methods known to the art for their use as candidate compounds, but they are preferably produced by biochemical methods, more preferably by recombinant expression in procaryotic or eucaryotic cells.

In step b) of the screening method the *Drosophila* cultures of step a) are incubated under incubation conditions, which generally allow normal *Drosophila* development, for an appropriate incubation period.

An appropriate incubation period is in particular the time period that is sufficient for wildtype *Drosophila* offsprings of the appropriate developmental stage of step a) to reach the developmental stage of pupariation.

Preferably the incubation of step b) can be performed for a time period of 4 to 12 days, preferably for 5 to 10 days, particularly for 7 days. The exact incubation period is generally determined by direct comparison with the time period that is required for wildtype *Drosophila* offsprings, which have the same age as the test offsprings and which are incubated in a parallel control culture, to reach the developmental stage of pupariation.

The incubation of step b) is generally performed at a temperature between 20 and 30 °C, particularly at 25°C and is preferably performed in a standard incubator. Furthermore it is preferred to keep the relative humidity at 50 to 70 %, particularly to 60 % during the incubation of step b).

Generally after one day of incubation larvae hatch from the laid eggs and start to perform their larval development in the culture medium that also contains the tested candidate compound for a DNA methyltransferase inhibitor.

If the tested candidate compound has the capacity to inhibit DNA methyltransferases, the larvae will survive and crawl out of the medium to pupariate on the wall of the culture vial.

In step c) those *Drosophila* cultures of step b) are identified that show the presence of mobile larvae or pupae. This identification can be performed by appropriate viability tests, but preferably is performed by visual inspection.

*Drosophila* cultures that contain a candidate compound or a mixture of candidate compounds with no effective methyltransferase inhibitor activity should lead to the phenotype of lethality in the larval stage. In the double transgenic offsprings of crossings of the parental strains *GawB(69B)* and *UAS-Dnmt3a* the phenotype of larval lethality is caused by the expression of the Gal4 gene and by the subsequent induction of the Dnmt3a gene by the Gal4 transcription activator at a time in development that is specific of the *GawB(69B)* transgene. In those aliquots that contain at least one candidate compound with an effective DNA methyltransferase inhibitor activity this phenotype of larval lethality is rescued by inhibition of the DNA methyltransferase Dnmt3a that is expressed at that defined time in development. Alterations of the DNA methylation pattern at that defined time in development are therefore prevented in *Drosophila* larvae of those cultures that contain an effective DNA methyltransferase inhibitor. Therefore those *Drosophila* larvae that had contact with an effective DNA methyltransferase inhibitor should survive and crawl out of the medium to pupariate at the inside of the culture vial. The presence of these surviving pupae can be easily detected by visual inspection. If the tested candidate compound shows no DNA methyltransferase inhibitor activity, the lethal modifications of the DNA methylation pattern by Dnmt3a is not prevented, the larvae will not proceed in their development and die in the medium.

Since the developing flies generally leave the medium for pupariation and pupariate on the inside of the culture vial, flies rescued by the contact with an effective inhibitor can be easily differentiated from flies that show a lethal phenotype before pupariation.
Therefore this simple test for the presence of pupae on the inside of the culture vials can serve as a time- and work-efficient experimental read-out for the aforementioned high-throughput screening method for the identification and characterization of DNA methyltransferase inhibitors, particularly of inhibitors of the DNA methyltransferase Dnmt3a.

Another crucial advantage of the double transgenic *Drosophila* strain *GawB(69B)* / *UAS-Dnmt3a* to be used in the above high-throughput screening method is the fact that early *Drosophila* development proceeds in the medium, whereby an intense contact with the candidate compound tested for DNA methyltransferase inhibitor activity dissolved in the medium is guaranteed. The later stages of *Drosophila* development however, particularly the stage of pupariation and the subsequent stages, take place outside the medium. Thus, intense contact between a candidate compound that is solved in the medium and the developing flies in later developmental stages can not be accomplished. Lethal phenotypes in later developmental stages are therefore not suitable to serve as a experimental read-out for the above screening method.

In a preferred embodiment of the screening method of the invention at least one negative and/or positive control experiment is performed.

Particularly, single transgenic offsprings of the *Drosophila* strain *GawB(69B)* leading to surviving, pupariating flies can be used as positive control experiment for the performance of a large scale screen. Single transgenic offsprings of the *Drosophila* strain *GawB(69B)* show no lethality at the larval or prepupal stage, independently on the presence or absence of a compound with DNA methyltransferase inhibitor activity.

Particularly, double transgenic offsprings of the transgenic *Drosophila* strain *GawB(69B)* and of a transgenic strain bearing a control construct in which the region encoding the catalytic domain of Dnmt3a has been deleted, as disclosed in in Lyko et al., Nature Genetics, 23, p. 363- 366 (1999), can be used as positive control experiment for the performance of a large scale screen.

Particularly, a test culture comprising double transgenic flies *GawB(69B)* / *UAS-Dnmt3a* but no candidate compound which leads to larval lethality can be used as negative control experiment for the performance of a large scale screen.

Another aspect of the present invention concerns the use of the offsprings, which are derived from a cross between the transgenic *Drosophila* strains *GawB(69B)* and *UAS-Dnmt3a,* for the identification and characterization of inhibitors of DNA methyltransferase, particularly of inhibitors of the DNA methyltransferases selected from the group consisting of Dnmt1, Dnmt2, Dnmt3a and Dnmt3b, preferably of inhibitors of the DNA methyltransferase Dnmt3a.

In an other aspect, the invention concerns the use of the phenotype of larval or prepupal lethality of the offsprings from a cross between the *Drosophila* strains *GawB(69B)* and *UAS-Dnmt3a* for the identification and characterization of inhibitors of DNA methyltransferases.

In an other aspect, the invention comprises the double transgenic *Drosophila* strain *GawB(69B)*/*UAS-Dnmt3a* that is derived from a cross of the single transgenic *Drosophila* strains *GawB(69B)* and *UAS-Dnmt3a.*

## Claims

1. A screening method for the identification and characterization of inhibitors of DNA methyltransferases comprising the following steps:
a) generation of at least one *Drosophila* culture which culture comprises
at least one offspring derived from a cross between flies of the *Drosophila* strains *GawB(69B)* and *UAS-Dnmt3a* in an appropriate developmental stage, a sufficient amount of Drosophila medium
and an appropriate concentration of at least one candidate compound for a DNA methyltransferase inhibitor,
b) incubation of the the *Drosophila* culture of step a) under incubation conditions which allow normal *Drosophila* development for an appropriate incubation period,
c) identification of those *Drosophila* cultures of step b) that show the presence of mobile larvae or pupae.

2. The screening method as claimed in claim 1, **characterized in that** the offspring of step a) is added to the culture in the developmental stage of the *Drosophila* egg.

3. The screening method as claimed in claim 1 or 2, **characterized in that** the incubation of step b) is performed for a time period that is sufficient for wildtype *Drosophila* offsprings of the appropriate developmental stage of step a) to reach the developmental stage of pupariation.

4. The screening method as claimed in any of claims 1 to 3, **characterized in that** the incubation temperature of step b) is from 20 to 30 °C, preferably 25 °C.

5. The screening method as claimed in any of claims 1 to 3, **characterized in, that** the incubation period of step b) is from 4 to 12 days, preferably 7 days.

6. The screening method as claimed in any of claim 1 to 5, **characterized in that** the DNA methyltransferase is selected from the group consisting of Dnmt1, Dnmt2, Dnmt3a and Dnmt3b, preferably Dnmt3a.

7. The screening method as claimed in claim 1 to 6, **characterized in that** the appropriate concentration of the candidate compound in the *Drosophila* culture of step a) is 100 µM to 10 mM.

8. The screening method as claimed in any of the claims 1 to 7, **characterized in that** the candidate compound of step a) is a compound is a library of molecules, a part or a mixture thereof.

9. The screening method as claimed in claim 8, **characterized in that** the library is composed of *small molecules.*

10. The screening method as claimed in claim 8, **characterized in that** the library is generated synthetically.

11. The screening method as claimed in claim 8, **characterized in that** the library is generated by biochemical methods, preferably by recombinant expression.

12. The screening method as claimed in any of the claims 1 to 11, **characterized in, that** the identification of those *Drosophila* cultures in step c) is performed by visual inspection.

13. The use of an offspring, which is derived from a cross between the *Drosophila* strains *GawB(69B)* and *UAS-Dnmt3a,* for the identification and characterization of inhibitors of DNA methyltransferases.

14. The use of claim 13, **characterized in, that** the DNA methyltransferase is selected from the group consisting of Dnmt1, Dnmt2, Dnmt3a and Dnmt3b, preferably Dnmt3a.

15. Transgenic *Drosophila* flies of the strain *GawB(69B)*/*UAS-Dnmt3a* derived from a cross between the *Drosophila* strains *GawB(69B)* and *UAS-Dnmt3a.*
